(19) Europäisches Patentamt
European Patent Office
Office européen des brevets

(11) Veröffentlichungsnummer: **0 213 340 B1**

(12) # EUROPÄISCHE PATENTSCHRIFT

(45) Veröffentlichungstag der Patentschrift: **02.05.91**

(51) Int. Cl.5: **C07C 51/255**, C07C 63/04, C07C 63/06, C07C 63/36, C07C 65/21, C07C 63/70

(21) Anmeldenummer: **86109524.8**

(22) Anmeldetag: **11.07.86**

(54) **Verfahren zur Herstellung von aromatischen Carbonsäuren.**

(30) Priorität: **16.08.85 DE 3529380**

(43) Veröffentlichungstag der Anmeldung: **11.03.87 Patentblatt 87/11**

(45) Bekanntmachung des Hinweises auf die Patenterteilung: **02.05.91 Patentblatt 91/18**

(84) Benannte Vertragsstaaten: **DE FR GB NL**

(56) Entgegenhaltungen:
**CH-A- 346 867**
**DE-A- 1 069 140**
**US-A- 2 005 183**
**US-A- 2 952 703**

(73) Patentinhaber: **HÜLS AKTIENGESELLSCHAFT**
**Paul-Baumann-Strasse 1**
**W-4370 Marl 1(DE)**

(72) Erfinder: **Feld, Marcel, Dr.**
**Im Lochgarten 56**
**W-5000 Köln 90(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 213 340 B1

**Beschreibung**

Die vorliegende Erfindung betrifft ein Verfahren zur Herstellung von aromatischen Carbonsäuren durch Oxidation von Acylaromaten in flüssiger Phase, vorzugsweise in essigsaurer Lösung, mit Sauerstoff bzw. einem Sauerstoff enthaltenden Gas.

Die bekannte Herstellung von aromatischen Carbonsäuren aus Alkylaromaten mit Cobalt-Katalysatoren nach z.B. der DE-OS 31 28 147.8 hat den Mangel, daß aromatische Monocarbonsäuren mit weiteren nach dem dortigen Verfahren oxidierbaren Substituenten nicht herstellbar sind. Für solche vielfach verwendeten Carbonsäuren fehlt ein einfach ausführbares Herstellverfahren. Es war bereits bekannt, daß bei der katalytischen Oxidation mit Luft an aliphatische Reste oder aromatische Rest gebundene Acylgruppen gemäb US-A 2005183 die zugehören Carbonsäuren und gemäß DE-A-1069140 Diacetylbenzol Terephthalsäure bilden.

Gegenstand der Erfindung ist das Verfahren zur Herstellung von aromatischen Carbonsäuren, gemäß Patentanspruch. Ein Acylaromat oder ein substituierter Acylaromat wird in Gegenwart einer im Reaktionsgemisch löslichen Mangan-(II)-Verbindung als Katalysator bei Temperaturen von 80 bis 200° C mit Sauerstoff bzw. einem Sauerstoff enthaltenden Gas oxidiert. Bevorzugte Manganverbindungen sind Mangan-II-acetat und Mangan-II-acetylacetonat.

Als Acyl wird die Gruppe

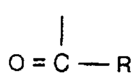

verstanden, worin R die Bedeutung Alkyl mit 1 bis 6 C-Atomen oder Benzyl hat, wobei auch weitere Reste R in Frage kommen, soweit nur das der Carbonylgruppe benachbarte C-Atom mindestens noch ein H-Atom aufweist.

Der entscheidende Vorteil des erfindungsgemäßen Verfahrens gegenüber anderen Oxidationsverfahren zur Herstellung von aromatischen Carbonsäuren, insbesondere gegenüber der durch eine Kombination von einer Kobalt-und einer Bromverbindung katalysierten Oxidation von Alkylaromaten mit Sauerstoff besteht darin, daß es die selektive Oxidation aryl-ständiger Acylgruppen neben anderen oxidierbaren Gruppen, wie beispielsweise Alkyl-, Alkoxy-, Benzyl-, Formyl- oder Hydroxymethylgruppen gestattet, welche vollständig oder zumindest größtenteils unverändert bleiben.

Die Selektivität des erfindungsgemäßen Verfahrens kommt sowohl dann zur Geltung, wenn ein Aromat neben einer Acylgruppe und ggf. einer oder mehrerer nicht oxidierbarer Gruppen, wie beispielsweise Halogen, Cyano-, Nitro-, Phenyl-, Phenoxy-, Carboxy-oder Carbalkoxygruppen zusätzlich eine oder mehrere der durch Sauerstoff oxidierbaren Gruppen enthält oder wenn eine Mischung aus einem Acylaromaten und einem Aromaten mit einem der oxidierbaren, aber verfahrensgemäß unverändert bleibenden, Substituenten zur Oxidation eingesetzt wird.

So kann unter den selektiven Oxidationsbedingungen beispielsweise ein Acetophenon neben Toluol zu Benzoesäure oxidiert werden, ohne daß dabei die Methylgruppe des Toluols in nachweisbarem Maße oxidativ angegriffen wird. Der selektive Oxidationsverlauf kann besonders am Beispiel einer Mischung von Acetophenon und einem substituierten Toluol, beispielsweise o-Chlortoluol, verdeutlicht werden:

Unter den erfindungsgemäßen Bedingungen entsteht dann praktisch ausschließlich Benzoesäure, nicht dagegen die substituierte Benzoesäure, beispielsweise o-Chlorbenzoesäure. Wird dagegen ein am Kern substituiertes Acetophenon, beispielsweise Chloracetophenon, neben Toluol unter den selektiven Bedingungen des erfindungsgemäßen Verfahrens oxidiert, dann entsteht praktisch ausschließlich die substituierte Benzoesäure, beispielsweise Chlorbenzoesäure neben unverändertem Toluol.

Beispiele für die selektive Oxidation einer arylständigen Acylgruppe von Aromaten, die neben der Acylgruppe auch noch weitere, durch Sauerstoff oxidierbare Substituenten enthalten, sind die Oxidation von Methoxyacetophenon, Methylacetophenon oder Acetyl-methylnaphthalin. Unter Verwendung eines Mangankatalysators werden in diesen Fällen bei Abwesenheit eines Kobalt-und Bromkatalysators in hohen Ausbeuten Anissäure bzw. Toluylsäure bzw. Methylnaphthoesäure erhalten.

Diese nach dem erfindungsgemäßen Verfahren durch selektive Oxidation herstellbaren aromatischen Carbonsäuren mit zusätzlichen oxidierbaren Substituenten, wie beispielsweise Toluylsäuren, Dimethoxy- und Trimethoxybenzoesäuren, haben u.a. als Ausgangsprodukte für pharmazeutische Wirkstoffe eine große Bedeutung und sind auf anderem Wege teilweise nur schwer zugänglich.

Entscheidend für das erfindungsgemäße Verfahren ist die Verwendung des Mangankatalysators, der in Form einer im Reaktionsmedium löslichen Mangan (II)-Verbindung, beispielsweise als Mangan (II)-acetat oder Mangan (II)-acetylacetonat, in Konzentration von ca. 0,001 bis 0,1, insbesondere von 0,01 bis 0,05 Gew.-Teilen (berechnet als Mangan) pro Gew.-Teil des Acylaromaten verwendet wird. Sofern der Aromat neben der Acylgruppe keine weiteren, durch Sauerstoff oxidierbaren Gruppen

enthält bzw. nicht zusammen mit einem anderen, oxidierbaren Aromaten zur Oxidation eingesetzt wird, kann neben dem Mangankatalysator auch noch eine Kobalt-und/oder Bromverbindung als zusätzlicher Katalysator verwendet werden. Wird jedoch eine selektive Oxidation ausschließlich der arylständigen Acylgruppe im Falle eines Acylaromaten mit weiteren oxidierbaren Substituenten oder neben einem anderen oxidierbaren Aromaten gewünscht, so wird die Oxidation vorzugsweise nur mit einem Mangankatalysator, zumindest aber ohne eine der beiden, für die Oxidation von Alkylaromaten so wirkungsvollen Komponenten einer Kobalt- und einer Bromverbindung neben dem Mangankatalysator durchgeführt.

Das erfindungsgemäße Verfahren wird bei erhöhten Temperaturen von ca. 80 bis 200° C durchgeführt. Die jeweils besonders geeignete Reaktionstemperatur ist außer vom speziellen Acylaromaten auch vom Lösungsmittel, der Katalysatorkonzentration und vom Sauerstoffpartialdruck abhängig. Bevorzugt wird mit Luft von Normaldruck bis ca. 80 bar, vorzugsweise von ca. 5 bis 50 bar bei Temperaturen von ca. 100 bis 180° C oxidiert.

Als Lösungsmittel bzw. Reaktionsmedien sind für die erfindungsgemäße Oxidation von Acylaromaten besonders aliphatische Carbonsäuren, vorzugsweise Monocarbonsäuren mit 2 bis 4 C-Atomen und insbesondere Essigsäure, gut geeignet. Allerdings können auch andere, unter den Reaktionsbedingungen hinreichend oxidationsbeständige Verbindungen, wie beispielsweise Wasser, Acetanhydrid oder bestimmte Aromaten als Lösungsmittel bzw. als Bestandteile einer Lösungsmittelmischung - vorzugsweise in Kombination mit Essigsäure - verwendet werden.

Bevorzugt wird jedoch in essigsaurer Lösung gearbeitet. Die Essigsäure kann dabei in reiner Form, wie auch in wässriger Verdünnung oder zusammen mit Acetanhydrid eingesetzt werden. Die Beispiele der Oxidation von Acetophenonen neben Toluol oder Chlortoluol zeigen, daß auch Aromaten zumindest als Bestandteile eines Lösungsmittelgemisches geeignet sind.

Acylaromaten als Ausgangsstoffe der Oxidation, insbesondere solche, die noch eine oder mehrere Alkyl- oder Alkoxygruppen am Kern tragen, sind durch Friedel-Crafts-Acylierung zugängig. Im allgemeinen werden dabei Monoacylaromaten erhalten, die deswegen im vorliegenden Verfahren als Ausgangsstoffe bevorzugt sind.

Sofern das Zielprodukt aufgrund einer zu hohen Löslichkeit nicht direkt aus dem Reaktionsgemisch durch eine übliche Fest-Flüssig-Trennung in befriedigender Ausbeute isolierbar ist, kann dem Reaktionsgemisch ein Lösungsmittel geringen Lösungsvermögens für das Zielprodukt, beispielsweise Wasser, zugesetzt oder aber das Reaktionsgemisch zunächst eingeengt, der Rückstand dann in einem derartigen Lösungsmittel aufgenommen und aus der so erhaltenen Suspension das Zielprodukt durch eine übliche Fest-Flüssig-Trennung isoliert werden. Ist dagegen das Zielprodukt wie im Falle der durch Oxidation von 2-Acetyl-6-methylnaphthalin erhältlichen 6-Methyl-2-naphthoesäure direkt aus dem Reaktionsgemisch, beispielsweise durch Filtration, isolierbar, so kann vorteilhaft das nach der Produktisolierung erhaltene, katalysatorhaltige und entsprechend der Löslichkeit auch noch Zielprodukt enthaltende Filtrat, ggf. nach Abtrennung bei der Oxidation entstandener Leichtsieder, wie beispielsweise Wasser oder Ameisensäure, als Reaktionsmedium für weitere Oxidationen des Acylaromaten verwendet werden. Durch eine derartige Wiederverwendung der Mutterlauge wird nicht nur der insgesamt erdorderliche Katalysatorbedarf gesenkt, sondern gleichzeitig auch die Ausbeute am Zielprodukt gesteigert.

Beispiel 1

Ein mit Rührer, Gaseinleitungsrohr, Temperaturfühler, Manometer und Druck-Rückflußkühler ausgestatteter, beheizbarer Autoklav aus Hastelloy C wurde mit 61,5 g Acetophenon, 63,5 g o-Chlortoluol, 500 g Essigsäure und 4 g Mangan (II)-acetat bei 140° C, 25 bar und einer Gasaustrittsgeschwindigkeit von 2 ltr. min. oxidiert. Das nach einer Reaktionszeit von 160 min. erhaltene Reaktionsgemisch wurde zur Trockne eingedampft, wobei das Destillat Essigsäure. Wasser und das o-Chlortoluol enthält. Der Destillationsrückstand wurde in 50 g heißer Essigsäure aufgenommen. Nach Zusatz von 1 ltr. Wasser wurden 52 g Benzoesäure (82.9% d.Th.) mit einer gaschromatographisch bestimmten Reinheit von 99.4% isoliert. Das Produkt enthielt bei einer Nachweisbarkeitsgrenze von 0.05 % keine o-Chlorbenzoesäure.

Beispiel 2

Unter den in Beispiel 1 beschriebenen Bedingungen wurde 100 g 4-Methoxy-acetophenon in einer Lösung von 10 g Mangan (II)-acetat in 400 g Essigsäure oxidiert. Der durch Einengen des Reaktionsgemisches erhaltene Rückstand (115 g) wurde in 800 g Wasser verrührt. Die Filtration ergab 95.7 g (94.8 % d. Th.) Anissäure (p-Methoxy-benzoesäure).

Beispiel 3

Analog Beispiel 1 wurde eine Mischung von

100 g 2-Acetyl-6-methylnaphthalin. 380 g Essigsäure, 20 g Wasser und 15 g Mangan (II)-acetat bei 140° C, 25 bar und einem Gasaustritt von 3 ltr./min. oxidiert. Nach einer Reaktionsszeit von 105 min. wurde unter Rühren auf Raumtemperatur abgekühlt, filtriert und der Filterkuchen mit 300 g 95 Gew.-%iger Essigsäure gewaschen. Nach dem Trocken wurden 70.3 g 6-Methyl-2-naphthoesäure (69.6 % d. Th.) mit einer gaschromatographisch bestimmten Reinnheit von 99.5 % erhalten. Die mit den Wasschfiltraten vereinigte Mutterlauge wurde unter Abdestillation von Wasser. Ameisensäure und Essigsäure, auf 380 g eingeengt. Nach Zusatz von 20 g Wasser 67.5 g 2-Acetyl-6-methylnaphthalinund 3.5 g Mangan (II)-acetat wurde erneut unter den zuvor beschriebenen Bedingungen oxidiert und aufgearbeitet. Mit 67.3 g wurde dann, bezogen auf die eingesetzen 67.3 g Acetyl-methylnaphthalin, eine Ausbeute an 6-Methyl-2-naphtheosäure von 98.9 % d. Th. erhalten.

Beispiel 4

Unter den in Beispiel 1 beschriebenen Bedingungen wurden 135 g Propiophenon in einer Lösung von 4 g Mangan (II)-acetat in 500 g Essigsäure oxidiert. Der durch Einengen des Reaktionsgemisches erhaltene Rückstand wurde in 100 g heißer Essigsäure aufgennommen. Nach Zusatz von 1.500 $cm^3$ Wasser wurden durch Filtration 86.0 g Benzoesäure (70 % d. Th.) isoliert.

Beispiel 5

Analog Beispiel 4 wurde eine Mischung von 100 g Desoxibenzoin, 370 g Essigsäure, 30 g Wasser und 4 g Mangan (II)-acetat oxidiert. Das Reaktionsgemisch wurde destillativ bis auf ein Gewicht von 210 g eingeengt, mit 1,2 l Wasser versetz, zum Sieden erhitzt und die heiße wässrige Lösung von dem Rückstand abdekantiert. Aus der abgekühlten wässrigen Lösung wurden durch Filtration 39.2 g Benzoesäure isoliert.

Beispiel 6

Analog Beispiel 1 jedoch bei 120° C und 30 bar. wurde eine Mischung von 60 g Acetophenon, 60 g Cumol, 500 g Essigsäure und 4 g Mangan (II)-acetat oxidiert und auch wie dort beschrieben aufgearbeitet. Beim Einengen des Reaktionsgemisches wurde ein Destillat mit über 10 % Cumol erhalten. Der aufgearbeitete Destillationsrückstand ergab 69.3 g Benzoesäure.

Beispiel 7

Analog Beispiel 1, jedoch bei 40° C, wurde eine Mischung von 60 g Acetophenon, 58 g Anisol, 500 g Essigsäure und 4 Mangan (II)-acetat oxidiert und, wie dort beschrieben aufgearbeitet. Es wurden 51.1 g Benzoesäure (83.5 % d. Th.) und ein Destillat mit über 10 % Anisol erhalten.

Beispiel 8

Analog Beispiel 1, wurde eine Mischung von 100 g 4-Chloracetophenon, 100 g Toluol, 400 g Essigsäure und 4 g Mangan (II)-acetatylacetonat oxidiert. Aus dem Reaktionsgemmisch, das zuvor mit den zum Spülen des Reaktionsgefäßes verwendeten 550 g Essigsäure vereint worden war, wurden durch Filtration 77.4 g (76.6 %) p-Chlorbenzoesäure isoliert.

Beispiel 9

Unter den in Beispiel 1 beschriebenen Bedingungen wurde eine Mischung von 50 g 4-Methylacetophenon, 400 g Essigsäure, 40 g Wasser und 5 g Manganacetat oxidiert. Das heiße Reaktionsgemisch wurde mit 160 g Wasser versetzt und unter Rühren auf Raumtemperatur abgekühlt. Durch Filtration wurden dann 25.3 g p-Toluylsäure (50.1 % d. Th.) isoliert. Das Produkt enthielt keine Terephthalsäure.

## Ansprüche

**1.** Verfahren zur Herstellung von aromatischen Monocarbonsäuren, dadurch gekennzeichnet, daß

a) substituierte Monoacylaromaten der Formel

Ar - C (0) - R

worin R Alkyl mit 1 bis 6 C-Atomen, Benzyl oder ein Rest bedeutet, worin das der Carbonylgruppe benachbarte C-Atom noch mindestens 1 H-Atom aufweist, und Ar einen aromatischen Rest mit mindestens einem Alkyl-, Alkoxy-, Benzyl-, Formyl- oder Hydroxymethyl-Substituenten darstellt oder

b) Mischungen von:

b1) substituierten Monoacylaromaten der

unter a) angegebenen Formel

Ar - C (O) - R

oder unsubstituierten Monoacylaromaten, mit:
b2) Aromaten, die mindestens einen Alkyl-, Alkoxy-, Benzyl-, Formyl- oder Hydroxymethyl-Substituenten aufweisen, in Gegenwart einer im Reaktionsgemisch löslichen Mangan (22)-Verbindung in Mengen von 0,001 bis 0,1 Gew.-Teilen Mangan pro Gewichtsteil Acylaromat als Katalysator und in Abwesenheit weiterer Katalysatoren bei Temperaturen von 80 bis 200 °c mit Sauerstoff bzw. einem Sauerstoff enthaltenden Gas oxidiert, werden wobei die an sich oxidierbaren Gruppen unverändert bleiben.

## Claims

**1.** Process for the production of aromatic monocarboxylic acids, characterised in that;
a)substituted monoacylaromatic compounds of the formula
Ar - C (O) - R
wherein R denotes alkyl with 1 to 6 C-Atoms, benzyl or a residue wherein the C-atom next to the carbonyl group has at least 1 H-atom, and Ar represents an aromatic residue with at least one alkyl, alkoxy, benzyl, formyl, or hydroxymethyl substituent or
b) mixtures of:
b1) substituted monoacylaromatic compounds of the formula indicated under a)
Ar - C (O) - R
or unsubstituted monoacylaromatic compounds, with
b2) aromatic compounds which have at least one alkyl, alkoxy, benzyl, formyl or hydroxymethyl substituent,
are oxidised at temperatures of 80 to 200 °C with oxygen on oxygen - containing gas in the presence of a manganese (II) compound soluble in the reaction mixture in amounts of 0.001 to 0.1 parts by weight of manganese per part by weight of acylaromatic compound as catalyst, and in the absence of further catalysts, with the oxidisable groups remaining unchanged.

## Revendications

**1.** Procédé de préparation d'acides monocarboxyliques aromatiques, caractérisé en ce que
a) des hydrocarbures aromatiques monoacylés substitués de formule

Ar - C (O) - R

dans laquelle R signifie un alkyle avec 1 à 6 atomes de carbone, un benzyle ou un reste où l'atome de C voisin du groupe carbonyle comporte encore au moins 1 atome d'H et Ar représente un reste aromatique avec au moins un substituant alkyle, alcoxy, benzyle, formyle ou hydroxyméthyle ou
b) des mélanges
b1) d'hydrocarbures aromatiques monoacylés substitués répondant à la formule indiquée sous a)

Ar - c (O) - R

ou d'hydrocarbures aromatiques monoacylés non substitués, avec
b2) des hydrocarbures aromatiques comportant au moins un substituant alkyle, alcoxy, benzyle, formyle ou hydroxyméthyle, sont oxydés avec de l'oxygène ou un gaz contenant de l'oxygène à des températures de 80 à 200 °C en présence d'un composé de manganèse(II) soluble dans le mélange réactionnel en tant que catalyseur, en des quantités de 0,001 à 0,1 partie en poids de manganèse par partie en poids d'hydrocarbure aromatique acylé et en l'absence d'autres catalyseurs, les groupes oxydables en tant que tels demeurant intacts.